# EUROPEAN PATENT SPECIFICATION

(11) **EP 2 391 370 B1**
(45) Date of publication and mention of the grant of the patent: **03.06.2015**
(21) Application number: 10706394.3
(22) Date of filing: 01.02.2010
(51) Int. Cl.: A61K 31/575, A61P 25/28

(54) **METHODS AND COMPOSITIONS FOR TREATING ALZHEIMER'S DISEASE**
VERFAHREN UND ZUSAMMENSETZUNGEN ZUR BEHANDLUNG VON ALZHEIMER-KRANKHEIT
PROCÉDÉS ET COMPOSITIONS DE TRAITEMENT DE LA MALADIE D'ALZHEIMER

(30) Priority: 02.02.2009 US 149100 P
(43) Date of publication of application: 07.12.2011
(73) Proprietor: Galmed Research and Development Ltd., 6473307 Tel-Aviv (IL)
(72) Inventor: GILAT, Tuvia, 64235 Tel Aviv (IL); ANGEL, Itzchak, 74045 Ness Ziona (IL)
(74) Representative: Becker Kurig Straus
(86) International application number: PCT/IL2010/000088
(87) International publication number: WO 2010/086864

(56) References cited:
- WO-A1-99/52932
- WO-A1-02/083147
- WO-A2-2006/050165
- JP-A- 2006 016 318
- JP-A- 2006 016 319
- RAMALHO ET AL: "Bile acids and apoptosis modulation: an emerging role in experimental Alzheimer's disease" TRENDS IN MOLECULAR MEDICINE, ELSEVIER CURRENT TRENDS, GB LNKD- DOI:10.1016/J.MOLMED.2007.12.001, vol. 14, no. 2, 22 January 2008 (2008-01-22), pages 54-62, XP022477479 ISSN: 1471-4914
- GONEN A ET AL: "Fatty acid bile acid conjugates inhibit atherosclerosis in the C57BL/6 mousemodel" PATHOBIOLOGY, KARGER, BASEL, CH LNKD- DOI:10.1159/000069332, vol. 70, no. 4, 1 April 2002 (2002-04-01), pages 215-218, XP009086624 ISSN: 1015-2008
- CHEN JING ET AL: "Retinoic acid isomers up-regulate ATP binding cassette A1 and G1 and cholesterol efflux in rat astrocytes: implications for their therapeutic and teratogenic effects.", THE JOURNAL OF PHARMACOLOGY AND EXPERIMENTAL THERAPEUTICS SEP 2011, vol. 338, no. 3, September 2011 (2011-09), pages 870-878, ISSN: 1521-0103

### SEQUENCE LISTING

<110> Galmed International Limited
<120> METHODS AND COMPOSITIONS FOR TREATING ALZHEIMER'S DISEASE
<130> GILAT/002 PCT
<150> 61/149100
   <151> 2009-02-02
<160> 2
<170> PatentIn version 3.5
<210> 1
   <211> 42
   <212> PRT
   <213> Homo sapiens
<400> 1
<210> 2
   <211> 770
   <212> PRT
   <213> Homo sapiens
<400> 2

## Description

### FIELD OF THE INVENTION

The present invention relates to the use of bile acid fatty acid conjugates and bile salt fatty acid conjugates (BAFACs also known as FABACs) for the prevention and treatment of brain diseases characterized by amyloid plaque deposits. In particular the present invention relates to compositions comprising FABACs for use in the prevention and treatment of Alzheimer's disease (AD).

### BACKGROUND OF THE INVENTION

Alzheimer's disease (AD) is a neurodegenerative disorder of the central nervous system leading to progressive dementia. The disease is characterized by progressive memory loss, behavioral and personality changes, and the decline of other higher cognitive functions. These losses are related to the death of brain cells and the breakdown of the connections between them. The course of this disease varies from person to person, as does the rate of decline. Approximately one out of ten people of age 65 and over suffer from mild to moderate dementia. On average, AD patients live for 8 to 10 years after they are diagnosed, though the disease can last for up to 20 years. At present, AD has no effective medical therapy.

Amyloid plaques are a major characteristic of AD. Plaques are dense, largely insoluble deposits of protein and cellular material outside and around the brain's neurons. Amyloid plaques develop first in areas of the brain used for memory and other cognitive functions. They consist of largely insoluble deposits of beta amyloid (Abeta; Aβ) a protein fragment of a larger protein called amyloid precursor protein (APP) intermingled with portions of neurons and with non-nerve cells such as microglia and astrocytes. It is not known whether amyloid plaques themselves constitute the main cause of AD or whether they are a by-product of the AD process. Given that increased Aβ generation, its aggregation into plaques, and the resulting neurotoxicity may lead to AD, it is of therapeutic interest to investigate conditions under which Aβ aggregation into plaques might be slowed down or even blocked.

Impaired cholesterol metabolism has been correlated with production of amyloid plaque in the central nerve system (CNS). Thus, the use of cholesterol lowering drugs (e.g., statins) for the treatment of AD and other amyloid plaque related diseases has been the focus of extensive research efforts. However, currently there is no consistent evidence to support cholesterol lowering drugs effect on amyloid formation in the CNS. Moreover, researches have announced that in two large clinical trials, the leading statins failed to show any effect against established AD and dementia. Therefore, there is still an unmet need for lowering cholesterol levels in the CNS.

The etiology and pathogenesis of AD are incompletely understood and may well be multifactorial. Even though strong evidence exists of a link between cholesterol and Alzheimer's Disease, it has not been possible to influence the course of the disease or prevent the disease by use of medications known to lower blood cholesterol. In other words patients given drugs approved for cholesterol reduction do not show diminished incidence or progression of AD.

Epidemiologic studies have shown a correlation between lipid and, particularly, cholesterol metabolism and AD. Retrospective studies comparing aged people with and without Alzheimer's disease, hypercholesterolemia was identified as a risk factor for Alzheimer's disease (Stozicka et al., Acta Virol. 2007; 51:205-22; Hartmann et al., J.Neurochem, 2007;103 Suppl 1:159-70; Kuller LH. Curr Atheroscler Rep. 2007:9:154-61; and Koudinova et al., Neurobiol Lipids 2003, 1, 6:27-33). Presence of the e-4 allele of apolipoprotein E (ApoE-4), a major brain lipoprotein, was found to be one of the strongest risk factors for AD (Cagnin et al., J.Neural. Transm Suppl. 2007 (72): 175-9; and Packard et al., J.Am.Geriatr Soc. 2007; 55:1777-85). Beta- as well as gamma-secretase activities, which produce beta amyloid by cleavage from the Amyloid Precursor Protein (APP), were more sensitive to cholesterol in patients with AD than in control subjects. Cholesterol added to neuronal cultures or cell lysates stimulated both secretase activities as well as the secretion of Amyloid beta. Depletion of cellular cholesterol had the opposite effects, reducing secretase activity and Abeta secretion (Xiong et al., Neurobiol.Dis.2007). These findings suggest that cholesterol homeostasis is impaired in the AD brain and that altered activity of relevant nuclear receptors may contribute to cholesterol retention and Amyloid beta production in the brain of AD patients. It was found that brains of AD patients had increased cholesterol retention compared with age-matched controls (Xiong et al., 2007). Cholesterol is believed to be a factor in neurotransmission and in the pathogenesis of amyloid plaques (the morphologic hallmark of AD) in AD and related diseases such as Cerebral Amyloid Angiopathy (CAA) (Koudinova et al., 2003).

Nevertheless, there is at present no established causal or causative relationship between serum cholesterol levels and the etiology or pathogenesis of Alzheimer's Disease. Furthermore, the use of well known cholesterol lowering drugs such as statins, have not proven to be correlated to reduced incidence or severity of AD or with any prognostic benefit. Some studies have reported decreased numbers of neurofibrillary tangles in the brains of patients with AD who were concomitantly receiving statins but these studies were flawed inasmuch as they are based on patients who happened to have received statins rather than controlled clinical studies (Li et al. Neurology 2007, 69, 878). Thus, while it has been speculated that certain types of cholesterol lowering drugs might benefit AD patients there is no consistent evidence to support this conjecture. Furthermore, a recent study assessed effect of statins on AD and dementia through evaluation of two doubleblind randomized placebo-controlled trials (RCT) with 26,340 participants. The study concluded that statins, given in late life to individuals at risk of vascular disease, have no effect in preventing AD or dementia (McGuinness et al. Cochrane Database Syst. Rev. 2009 Apr 15;(2):CD003160).

US Pat. No. 6,080,778 discloses methods of preventing Alzheimer's disease by lowering blood cholesterol levels. US Pat. No. 6,440,387 discloses methods of predicting an individual's risk of developing Alzheimer's disease by measuring blood cholesterol level. These patents disclose only treatments for lowering blood cholesterol and do not deal with cholesterol metabolism within the brain.

Fatty Acid Bile Acid conjugates or Fatty Acid Bile Salt Conjugates (FABACs; synonym BAFACs) affect lipid, and in particular cholesterol, metabolism in the body. They are believed to lower cholesterol concentration in blood (Leikin-Frenkel et al., Arch.Biochem.Biophys. 2008, 47:63-71), increase sterol (cholesterol and its major metabolite, bile acids) output in the feces (Leikin-Frenkel et al., Biochem. Soc. Trans. 2004; 32:131-133), and reduce body cholesterol stores by enhancing CYP7A1 activity. This enzyme, which converts cholesterol to bile acids, is the main catabolic pathway, handling around 50% of body cholesterol. FABACs also reduce the activity of HMGCoA, the enzyme responsible for endogenous cholesterol synthesis in the liver (Leikin-Frenkel et al., 2008), stimulate cholesterol efflux from cells in an ABCA1-dependent manner, (Goldiner et al., Biochem J. 2006; 396(3):529-36). The transport of cellular cholesterol, via ABCA1, to HDL, to the Liver and its subsequent excretion via the bile into the intestine with subsequent loss in the feces is termed Reverse Cholesterol Transport. This pathway, initiated by ABCA1 and FABACs, is a major pathway for net cholesterol loss from the body. In addition, Fabacs reduce liver fat levels and gallstones (Gilat et al., Hepatology 2003; 38: 436-442; and Gilat et al., Hepatology 2002; 35: 597-600).

The known uses and indications for FABACs include treatment of gallstones, and reduction of arteriosclerosis as disclosed in US Patents 6,384,024, 6,395,722, 6,589,946 to one of the present inventors. More recently, these and additional types of bile salt fatty acid conjugates have been disclosed as useful for treatment of fatty liver, hyperglycemia and elevated serum cholesterol levels WO 2002/083147. The contents of US 6,384,024, 6,395,722 and 6,589,946 and WO 2002/083147 are incorporated herein as if set forth in their entirety.

Fatty Acid Bile Acid Conjugates (FABACs) lower cholesterol concentration in blood and increase sterol output in the feces. Thus, FABACs have been disclosed as useful for the treatment of several diseases resulting from high cholesterol levels in the blood or bile (e.g., gallstones, arteriosclerosis).

JP 2006 016318 relates to a pro-drug for improving the migration of 2-propyloctanoic acid to the brain, and being useful in the treatment of neurodegenerative diseases. The prodrug comprises a cholic acid residue linked via a spacer to a fatty acid having 11 carbon atoms.

JP 2006 016319 refers to the problem of reducing the stimulation of (blood) vessels by administration of 2-propyloctanoic acid. For solving this problem a pro-drug of 2-propyloctanoic acid which comprises a spacer and a fatty acid having 11 carbon atoms is suggested.

Ramalho et al. ("Bile acids and apoptosis modulation: an emerging role in experimental Alzheimer's disease"; Trends in mMolecular Medicine, Elsevier Current Trends, GB LNKD-DOI:10.1016/J.MOLMED.2007.12.001, Vol. 14, No. , January 22, 2008, p. 54-62) summarize the role of apoptosis in Alzheimer's disease and report about two endogenous bile acids that act as inhibitors of apoptosis, i.e. ursodeoxycholic acid (UDCA) and tauroursodeoxycholic acid (TUDCA).

WO 2006/050165 pertains to the treatment of neurodegenerative diseases and in particular amyotrophic lateral sclerosis (ALS), also known as Lou Gehrig's disease. The inventors suggest a solution comprising a bile acid (e.g. cholic acid), an aqueous soluble starch conversion product or an aqueous soluble non-starch polysaccharide.

Nowhere in the art is it disclosed or suggested whether FABACs can cross the blood brain barrier and whether the effects of FABACs can be exerted on brain cells, particularly on astrocytes and glial cells.

None of the above references discloses or suggests that FABACs are able to affect brain cholesterol metabolism. Moreover, nowhere in the prior art is it taught or suggested that FABACs could be useful for the treatment or prevention of deterioration of subjects at risk for Alzheimer's disease, or other brain diseases characterized by amyloid deposits.

There are currently no effective therapies for treating, preventing and, more importantly, reversing the impairment of central nervous system function once a degenerative cascade begins. Thus, there remains an unmet medical need for new therapeutic modes of treating Alzheimer's disease, and related brain diseases characterized by amyloid deposits.

### SUMMARY OF THE INVENTION

The present invention relates to methods for treatment, prevention, and inhibition of progression of Alzheimer's disease (AD), and other brain diseases characterized by amyloid plaque deposits, comprising administration to a subject of a bile acid or bile salt fatty acid conjugate (FABAC; synonym "BAFAC").

It is disclosed herein for the first time that FABACs increase cholesterol efflux from human astrocyte brain cells. Cholesterol efflux from brain cells, particularly astrocytes, physiologically mediated by apolipoprotein E (ApoE), is widely agreed to be of major importance in reducing amyloid deposition. Decreased cholesterol efflux enhances amyloid deposition while increased cholesterol efflux diminishes amyloid deposition. Unexpectedly, the FABACs were more potent in increasing cholesterol efflux than brain ApoE. Thus, the findings presented herein unexpectedly demonstrate that FABACs are potential therapeutic agents for Alzheimer's Disease and amyloid-related disorders in the brain.

As disclosed herein for the first time, FABACs cross the blood-brain barrier in animals, even though peak serum levels are not high and decline rapidly after a single dose in animals. In humans, unlike experimental animals, blood levels of Aramchol (a conjugate of cholic acid with arachidic acid) remain high for over 24 hours after a single administration as exemplified herein below. It will be understood by those skilled in the art that daily dosing in humans will further increase plasma levels. The combination of all these factors enables a continuous exposure of the brain to the therapeutic effects of the FABACs.

In one aspect, the present invention provides a pharmaceutical composition comprising as an active ingredient a bile acid fatty acid conjugate or bile salt fatty acid conjugate (FABAC), for use in the treatment, prevention, or inhibition of progression of a brain disease characterized by amyloid plaque deposits, wherein said FABAC has the formula II: W - X - G (II), wherein G represents a bile acid or bile salt; W represents one or two saturated or unsaturated fatty acids having 14-22 carbon atoms; and X represents an NH bonding member, and wherein said brain disease is selected from the group consisting of Alzheimer's disease, Down's syndrome, mild cognitive impairment, senile dementia and cerebral amyloid angiopathy.

According to one embodiment, the brain disease is Alzheimer's disease.

According to another embodiment, the composition for use comprises two fatty acids wherein at each occurrence W is independently a saturated or unsaturated fatty acid having 14-22 carbon atoms.

Preferably, in the composition for use the fatty acids are independently selected from the group consisting of behenylic acid, arachidylic acid, stearic acid, and palmitic acid. Alternatively, the bile acid is selected from the group consisting of cholic acid, ursodeoxycholic acid, chenodeoxycholic acid, deoxycholic acid, lithocholic acid, and derivatives thereof. Also alternatively, the bile acid or bile salt is conjugated to an amino acid.

According to another embodiment, in the composition for use the FABAC is selected from the group consisting of 3-beta-stearoyl-amido,7α,12α-dihydroxy-5-beta-cholan-24-oic acid and 3-beta arachidylamido,7α,12α-dihydroxy-5-beta-cholan-24-oic acid. Alternatively, the composition further comprises an inhibitor of cholesterol synthesis.

In another aspect the present invention provides a bile acid fatty acid conjugate or bile salt fatty acid conjugate (FABAC) for use in treating, preventing, or inhibiting progression of a brain disease, said brain disease being characterized by amyloid plaque, wherein said FABAC has the formula II: W - X - G (II), wherein G represents a bile acid or bile salt; W represents one or two saturated or unsaturated fatty acids having 14-22 carbon atoms; and X represents an NH bonding member, and wherein said brain disease is selected from the group consisting of Alzheimer's disease, Down's syndrome, mild cognitive impairment, senile dementia and cerebral amyloid angiopathy.

According to one embodiment, the brain disease is Alzheimer's disease.

According to another embodiment, the FABAC comprises two fatty acids wherein at each occurrence W is independently a saturated or unsaturated fatty acid having 14-22 carbon atoms.

According to yet another embodiment, the fatty acids are independently selected from the group consisting of behenylic acid, arachidylic acid, stearic acid, and palmitic acid. Alternatively, the bile acid is selected from the group consisting of cholic acid, ursodeoxycholic acid, chenodeoxycholic acid, deoxycholic acid, lithocholic acid, and derivatives thereof. Also alternatively, the bile acid or bile salt is conjugated to an amino acid. Also alternatively, the FABAC is selected from the group consisting of 3-beta-stearoyl-amido,7α,12α-dihydroxy-5-beta-cholan-24-oic acid and 3-beta arachidylamido,7a,12a-dihydroxy-5-beta-cholan-24-oic acid.

According to another embodiment, the FABAC is administered in a pharmaceutical composition that further comprises an inhibitor of cholesterol synthesis.

According to yet another embodiment, the bile acid fatty acid conjugate or bile salt fatty acid conjugate (FABAC) is used in the treatment, prevention, or inhibition of progression of a brain disease characterized by amyloid plaque deposits, wherein the brain disease is selected from the group consisting of Alzheimer's disease, Down's syndrome, mild cognitive impairment, senile dementia and cerebral amyloid angiopathy.

Other objects, features and advantages of the present invention will become clear from the following description and drawings.

### BRIEF DESCRIPTION OF THE FIGURES

**Figure 1** shows ¹⁴C-Aramchol radioactivity in the brain of male Wistar rats after oral administration.
**Figure 2** depicts Aramchol plasma levels after oral administration in normal human volunteers (n=12) at 4 dose levels.
**Figure 3** shows Aramchol effect on Cholesterol efflux from the human Astrocyte cell line CCFSTTG1 (*P= 0.0003 vs Medium; **P<0.0002 vs Medium; ***P=0.00001 vs Medium).
**Figure 4** shows Steamchol effect on Cholesterol efflux from the human Astrocyte cell line CCFSTTG1 (*P= 0.0003 vs medium; **P=0.0006 vs medium; ***P=0.0001 vs medium)
**Figure 5** shows ApoE4 effect on Cholesterol efflux from the human Astrocyte cell line CCFSTTG1 (*P= 0.0012 vs medium).
**Figure 6** shows ApoE3 effect on Cholesterol efflux from human Astrocyte cell line CCFSTTG1 (P= 0.0074 vs medium).
**Figure 7** is a graph comparing the Cholesterol efflux induced by FABACs and Apolipoproteins in human Astrocyte cell line CCFSTTG1 (* P= 0.0024 vs steamchol; **P=0.0047 vs steamchol; *** P<0.013 vs Apoliproteins; **** P<0.014 vs Apoliproteins).

### DETAILED DESCRIPTION OF THE INVENTION

The present invention relates to the field of treatment, prevention, and inhibition of progression of Alzheimer's disease, and other brain diseases characterized by amyloid plaque deposits, particularly comprising administration to a subject a bile acid-fatty acid conjugate or bile salt-fatty acid conjugate (FABAC).

Impaired cholesterol metabolism (e.g., impaired cholesterol efflux from brain cells to the extracellular fluid), has been correlated with production of amyloid plaque in the CNS. However, the use of cholesterol lowering drugs (e.g., statins) for treating or preventing AD and other amyloid plaque related diseases have not been shown to be effective. Therefore, there is still an unmet need for lowering cholesterol levels in the CNS.

While FABACs have been disclosed as being useful in lowering cholesterol concentration in blood, nowhere in the art is it disclosed or suggested that FABACs may lower cholesterol concentration in the CNS. As demonstrated herein, FABACs unexpectedly cross the blood brain barrier and remove cholesterol from glial brain cells and thus are beneficial in the treatment and prevention of brain diseases characterized by amyloid deposits (e.g., Alzheimer's disease).

As exemplified herein below, FABACs induce cholesterol efflux from a human astrocyte cell line in a dose response effect. Surprisingly, Steamchol (a conjugate of stearic and cholic acids) was more effective than Aramchol (a conjugate of cholic acid with arachidic acid), as opposed to what was previously found in fibroblasts. FABACs had a greater effect on cholesterol efflux from human astrocytes, than the human Apolipoproteins ApoE3 and ApoE4. While Steamchol and Aramchol showed maximal activity at a concentration of 2 µg/ml and 5-10 µg/ml, respectively, cholesterol efflux induced by lipoproteins increased significantly only at concentrations of 10 and 20 µg/ml. Surprisingly, even at these concentrations the effect of the apolipoproteins was not higher than that of the FABACs.

Without wishing to be bound by any theory or mechanism of action, FABACs may work by inducing egress of cholesterol (i.e., cholesterol efflux) from cells in the brain. In another embodiment, FABACs having removed the cholesterol from the cells to the extracellular fluid, thereby induce egress of cholesterol from cerebrospinal fluid (CSF) to the bloodstream, where-after they are eliminated from the body, e.g. in the feces. In another embodiment, FABACs decrease the amount of amyloid produced in a brain cell. In another embodiment, FABACs remove (e.g., export) amyloid from brain cells to the extracellular fluid. Each possibility represents a separate embodiment of the present invention.

Thus, FABACs are potential therapeutic agents for Alzheimer's Disease, and other brain diseases characterized by amyloid plaque deposits, beneficially affecting cholesterol metabolism and transport both at the systemic level and in the brain.

It has been reported that stimulation of liver X receptor (LXR) by synthetic agonists is beneficial to AD (Koldamova R. and Lefterov I, Current Alzheimer Research 2007, 4:171-178). Agonists of LXR, but also of retinoid X receptor (RXR) or peroxisome proliferator-activated receptors (PPAR), stimulated cholesterol efflux to HDL, reduced cellular cholesterol and beta/gamma secretase activities in N2a cells (Xiong et al., 2007). Stimulation of LXR stimulates multiple genes, among them SREBP, SCD1, ABCA1, as well as apolipoproteins. Thus, it is not possible to determine which of the many LXR stimulated genes, or combination thereof, are responsible for its beneficial activities on AD models including on the reduction of Abeta deposition. Moreover, agonists of LXR induce severe Fatty Liver Disease making them unsuitable, in their present form, to act as therapeutic agents. Among the many LXR stimulated genes and effector molecules the ATP-binding cassette transporter (ABCA1) received much attention. ABCA1 promotes efflux of cholesterol and phospholipids from intracellular compartments to extracellular cholesterol acceptors. ABCA1 gene deletion in mice increased Abeta (Amyloid Beta) deposition. Following LXR stimulation, ABCA1 activity was indeed up-regulated together with the activity of many other genes and Abeta deposition was decreased. However, it was not possible to pinpoint which gene, combination of genes, or which particular effect was responsible for the decrease in Abeta deposition, as almost all studies used LXR agonists. Thus at present it has not been demonstrated that ABCA1 stimulation will be beneficial in treating or preventing AD.

FABACs have no effect on LXR, stimulatory or inhibitory. Conversely, the two major effects of FABACs, stimulation of ABCA1 activity with concomitant inhibition of SCD1 activity (both genes stimulated physiologically by LXR) allow a clear separation of the general effects of LXR from the specific effects of ABCA1. By inhibiting the SCD1 liver lipogenic enzyme, FABACs reduce liver fat both in a preventive and therapeutic way (Gilat et al., 2003; and Leikin Frenkel et al. Europ.J.Gastroenter.Hepatol.2008). Several FABAC molecules (conjugates of cholic acid with C16, C18, C20 and C22 Fatty Acids) were shown to increase ABCA1 activity in fibroblasts, enhancing cholesterol efflux from fibroblasts to ApoA1 and hence to HDL inducing a net cholesterol loss from these cells (Goldiner et al., 2006). No where in the art is it disclosed or suggested whether the effects of FABACs are exerted on brain cells, particularly on astrocytes and glial cells.

Disclosed is a pharmaceutical composition comprising a bile acid fatty acid conjugate or bile salt fatty acid conjugate (FABAC), for the treatment, prevention, or inhibition of progression of a brain disease characterized by amyloid plaque deposits.

Disclosed is also a method for treating, preventing, or inhibiting progression of a brain disease characterized by amyloid plaque deposits in a subject in need thereof, the method comprising the step of administering to the subject therapeutically effective amount of a FABAC, thereby treating, preventing, or inhibiting progression of a brain disease characterized by amyloid plaque deposits in a subject. Disclosed is also a pharmaceutical composition comprising a FABAC is administered. Each possibility represents a separate embodiment of the present invention.

Also disclosed is use of a bile acid fatty acid conjugate or bile salt fatty acid conjugate (FABAC) in the preparation of a medicament for treatment, prevention, or inhibition of progression of a brain disease characterized by amyloid plaque deposits.

The brain disease treated by the methods and compositions disclosed is, in another embodiment, Alzheimer's disease. In another embodiment, the brain disease is any other brain disease characterized by amyloid plaque deposits. Each possibility represents a separate embodiment of the present invention.

"FABAC" (synonym BAFAC) as used herein, refers to conjugates of the formula W - X - G, wherein G represents a bile acid or bile salt radical, W represents one or two fatty acid radical(s) having 6-22 carbon atoms, and X represents a bonding member (e.g. (a non-limiting list of examples) NH, P, S, O, a direct C=C or C-C bond, etc.) between said bile acid or bile salt radical and the fatty acid radical(s). The bond can be any other solid bond that is not substantially deconjugated during digestion and absorption, with the proviso that an ester bond is not suitable (these are easily degraded). Non-limiting examples of bile acids are cholic acid, ursodeoxycholic acid, chenodeoxycholic acid, deoxycholic acid, lithocholic acid, and their derivatives. FABACs are known in the art, and are described, for example, in US Patents 6,384,024, 6,395,722, and 6,589,946, and in Gilat T et al. Gut 2001; 48(1):75-9, the contents of which are incorporated herein by reference. Other definitions of FABACs specify 8-22 carbon atoms, 14-22 carbon atoms or 18-22 carbon atoms in the fatty acid radical(s).

A non-limiting general structure of FABACs is set forth below. A bile acid is conjugated (e.g. using an amide bond, for example at position 3) with 1-2 fatty acids of any of a number of chain lengths.

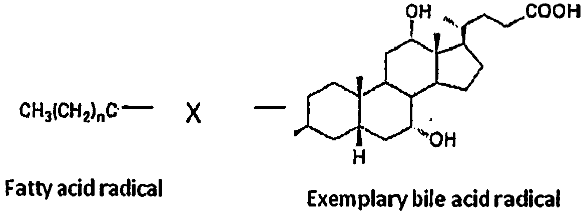

An additional non-limiting exemplary FABAC structure is set forth below

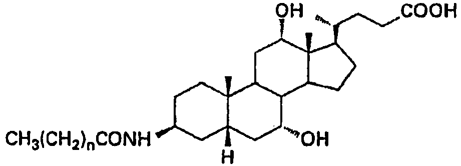

An example of a FABAC is 3β-arachidylamido-7α,12α, dihidroxy 5β-cholan-24-oic acid (Arachidyl Amido Cholanoic Acid; an amide conjugate of cholic acid with arachidic acid; also known as "Aramchol" or "C20 FABAC").

A FABAC may have the formula II:

W - X - G (II)

wherein G represents a bile acid or bile salt; W represents one or two saturated or unsaturated fatty acids having 6-22 carbon atoms; and X represents a bonding member or a direct C-C or a C=C bond, with the proviso that the bond is other than an ester bond.

A FABAC may have the formula II:

(W - X -)n G (II)

wherein G represents a bile acid or bile salt; W represents a fatty acid having 6-22 carbon atoms; X represents a bonding member comprising a heteroatom or a direct C-C or C=C bond, with the proviso that the bond is other than an ester bond; and n is an integer 1 or 2.

n may be 1. n may also be 2, and at each occurrence W is independently a fatty acid having 6-22 carbon atoms and X is independently a bonding member comprising a heteroatom or a direct C-C or C=C bond, with the proviso that the bond is other than an ester bond.

The bile acid or bile acid radical of the FABAC may be selected from the group consisting of cholic acid, ursodeoxycholic acid, chenodeoxycholic acid, deoxycholic acid, lithocholic acid, and derivatives thereof.

Each type of bile acid, bile salt, or radical thereof represents a separate embodiment of the present invention.

The term "radical" as used herein means a chemical moiety comprising one or more unpaired electrons.

The bile acid or bile salt may be conjugated (e.g. at position 24) to an amino acid. The amino acid may be selected from the group consisting of glycine. In another embodiment, the bile or bile salt is conjugated to taurine (2-aminoethanesulfonic acid), a derivative of cysteine.

The FABAC may have one fatty acid. The fatty acid may be saturated. The fatty acid may be unsaturated, mono-unsaturated, or poly-unsaturated.

The FABAC may have two fatty acids. The conjugation of each fatty acid radical to the bile acid nucleus may be at two positions selected from the 3, 7, 12 and 24 positions of the bile acid nucleus. The conjugations may be at position 3 and 7 of the bile acid nucleus.

Thus, a FABAC may have the formula:

(W - X -)₂ G

wherein G represents a bile acid or bile salt; W independently represents a saturated or unsaturated fatty acid having 6-22 carbon atoms; and X independently represents a bonding member comprising a heteroatom or a direct C-C or a C=C bond, with the proviso that an ester bond is not suitable. The fatty acids may be saturated, unsaturated, mono-unsaturated, or poly-unsaturated. A conjugated fatty acid may be utilized. Alternatively, an un-conjugated fatty acid one fatty acid may be unsaturated and the other may be saturated.

The fatty acid(s) or fatty acid radical(s) of the FABAC may be independently selected from the group consisting of behenylic acid, arachidylic acid, stearic acid, and palmitic acid. Linoleic acid may be utilized, or, a conjugated linoleic acid or a conjugated linoleic acid isomer.

The term "conjugated fatty acid", also known as "CFA", refers to polyunsaturated fatty acids in which at least one pair of double bonds are separated by only one single bond.

The fatty acid may be a short-chain fatty acid. The chain length may be 6-8 carbons. The fatty acid may be a medium-medium chain fatty acid and the chain length is 8-14 carbons or 14-22 carbons or 16-22 carbons. Any other fatty acid length known in the art may be utilized.

The bonding member of the FABAC may be selected from the group consisting of NH, P, S, O, or a direct C-C or C=C bond. The bond between the bile acid or bile salt radical and the fatty acid radical(s) may be in the beta configuration or in the alpha configuration. The bonding member may be a solid bond that is not substantially deconjugated during digestion and absorption in the gastrointestinal tract of the subject to which the composition is administered. The bonding member may not be substantially decomposed in the first 10, preferably 12 hours, following absorption in the gastrointestinal tract of the subject to which the composition is administered. In particular the term "substantially" refers to at least 70%, preferably at least 75% more preferably at least 80% of the FABAC administered.

"Direct C-C bond" may refer to a C-C (single) bond or to a C=C (double) bond. More than one bond may be utilized.

In general, the term "heteroatom" includes atoms of any element other than carbon or hydrogen, preferred examples of which include nitrogen, oxygen, sulfur, and phosphorus.

The conjugation of the bile acid or bile salt radical with the fatty acid radical may take place at various positions of the bile acid. The conjugation of the bile acid or bile salt radical with the fatty acid radical may be performed in a position of the bile acid nucleus selected from 3, 6, 7, 12 and 24. It is understood that when the bile acid is conjugated with an amino acid at position 24 of the bile acid nucleus, the conjugation with the fatty acid radical cannot be performed at said position. The conjugation may be performed in position 3 of the bile acid nucleus.

The FABAC may be selected from:
3β-behenylamido-7α, 12α -dihydroxy-5β -cholan-24-oic acid,
3β -arachidylamido-7α, 12α -dihydroxy-5β -cholan-24-oic acid,
3β -stearylamido-7α, 12α -dihydroxy-5β -cholan-24-oic acid,
3β -palmitylamido-7α, 12α -dihydroxy-5β -cholan-24-oic acid,
3β -myristylamido-7α, 12α -dihydroxy-5β -cholan-24-oic acid, and
N-(-carboxymethyl)-3β -stearylamido-7α, 12α -dihydroxy-5β -cholane-24-amide. Each possibility represents a separate embodiment of the present invention.

The FABAC may be 3β-stearoylamido-7α,12α- dihydroxy-5β-cholan-24-oic acid.

The conjugated linoleic acid isomer may be another conjugated linoleic acid isomer known in the art. Conjugated linoleic acid isomers are described, for example, in United States Patents 5,760,083 and 5,770,247 and United States Patent. Applications 2001/0031308, 2002/0049346, 2003/0225295, 2004/0018225, 2004/0087512, and 2006/0041017, and in Ha YL et al 1990 (Inhibition of benzo(a)pyrene-induced mouse forestomach neoplasia by conjugated dienoic derivatives of linoleic acid. Cancer Res. 1990 Feb 15;50(4):1097-101); Ha YL 1987 (Anticarcinogens from fried ground beef: heat-altered derivatives of linoleic acid. Carcinogenesis. 1987 Dec;8(12):1881-7); and Choi et al, Regulation of stearoyl-CoA desaturase activity by the trans-10,cis-12 isomer of conjugated linoleic acid in HepG2 cells. Biochem Biophys Res Commun. 2001 Jun 15;284(3):689-93). Preferably, the conjugated linoleic acid isomer is a conjugated dienoic linoleic acid derivative.

FABACs as described herein include pharmaceutically acceptable salts, derivatives and prodrugs. Methods for preparing FABACs and salts, derivatives and prodrugs of FABACs are well known in the art, and are further described in US 6,384,024, 6,395,722 and 6,589,946 and WO 2002/083147.

As used herein, the term "bile acid derivative" includes bile acid salts with their pharmaceutically acceptable bases or acids as well as their diastereoisomeric and enantiomeric forms.

### Abeta and amyloid plaque deposits

As used herein, the term "amyloid-beta" is interchangeable with the terms "amyloid-beta peptide", "Abeta," and "Aβ," and comprises a peptide fragment of the amyloid precursor protein (APP), as described in Selkoe, D. J., "Alzheimer's Disease: Genes, Proteins, and Therapy" Physiol. Rev. 87:741-766, 2001, which is hereby incorporated by reference herein. The term Abeta includes naturally occurring sequences derived from the carboxy-terminal region of APP (e.g., Aβ 1-28, Aβ 1-40 and Aβ 1-42) and amino-terminal truncations of Abeta. The amino acid sequence of human amyloid beta 1-42 is set forth in SEQ ID NO: 1 (DAEFRHDSGYEVHHQKLVFFAEDVGSNKGAIIGLMVGGVVIA). The amino acid sequence of human APP is set forth in SEQ ID NO: 2 (Accession No.: GI:4502167).

The term Abeta further includes peptides that are at least 80% identical (such as at least 90%, 95%, 98%, 99%) to Abeta 1-40 or at least 80% identical (such as at least 90%, 95%, 98%, 99%) to Abeta 1-42 or at least 80% identical (such as at least 90%, 95%, 98%, 99%) to Abeta 1-28. Abeta also includes monomeric and oligomeric forms of the peptide.

Amyloid can be identified by its amorphous structure, eosinophilic staining, changes in thioflavin fluorescence, and homogeneous appearance. In one embodiment, the term "amyloid" is used herein to refer to a substance that contains Aβ.

As used herein, the term "amyloid plaque deposits" refers to insoluble protein aggregates formed by the accumulation of amyloid peptides, such as Aβ42. In accordance with the present invention, the term "amyloid plaque deposits" relates to deposits in the central nerve system CNS (e.g., brain) of a subject. As used herein, the term "central nervous system" or CNS means the brain and spinal cord of a subject.

The term "amino acids" refers to both the naturally occurring amino acids and other unnaturally amino acids including both optically active (D and L) forms as well as racemic derivatives. As contemplated herein, the amino acids may be conjugated to the bile acid or bile salt by forming an amide bond between the carboxyl group of the bile acid (or bile salt) and the amino group of the amino acid. The naturally occurring amino acids are, e.g., glycine, alanine, valine, leucine, isoleucine, serine, methionine, threonine, phenylalanine, tyrosine, tryptophan, cysteine, proline, histidine, aspartic acid, asparagine, glutamic acid, glutamine, γ- carboxyglutamic acid, arginine, ornithine and lysine. Examples of unnatural α-amino acids include taurine (i.e., 2-aminoethanesulfonic acid), α-aminoisobutyric acid, α-aminobutyric acid, γ-aminobutyric acid, citrulline, N-methyl derivatives of any of the natural amino acids listed above (e.g., N-methyl-alanine, N-methyl-glycine (sarcosine), N-methyl-glutamic acid, N-methyl serine, N-methyl leucine, N-methyl isoleucine, N-methyl phenylalanine and N-methyl aspartate), homocitrulline, homoproline, homoserine, hydroxyproline, norleucine, 4- aminophenylalanine, 4-halo phenyl alanine (e.g., 4-fluoro, bromo, chloro or iodo phenylalanine wherein), 4-nitro phenylalanine, statine, hydroxy lysine, kynurenine, 3- (2'-naphthyl)alanine, 3-(r-naphthyl)alanine, methionine sulfone, (t- butyl)alanine, (t- butyl)glycine, 4-hydroxyphenylglycine, aminoalanine, phenylglycine, vinylalanine, propargyl-gylcine, 1 ,2,4-triazolo-3 -alanine, thyronine, 6-hydroxytryptophan, 5- hydroxytryptophan, 3 - hydroxy kynurenine, 3-aminotyrosine, trifluoromethyl-alanine, 2- thienylalanine, (2-(4-pyridyl)ethyl)cysteine, 3,4-dimethoxy-phenylalanine, 3-(2'- thiazolyl)alanine, ibotenic acid, 1 -amino- 1-cyclopentane-carboxylic acid, 1 -amino- 1- cyclohexanecarboxylic acid, quisqualic acid, 3-(trifuoromethylphenyl)alanine, (cyclohexyl)glycine, thiohistidine, 3-methoxytyrosine, elastatinal, norleucine, norvaline, alloisoleucine, homoarginine, thioproline, dehydroproline, hydroxyproline, homoproline, α-amino-n-butyric acid, cyclohexylalanine, 2-amino-3-phenylbutyric acid, β-2- and 3- thienylalanine, β-2- and 3-furanylalanine, β-2-, 3- and 4-pyridylalanine, β- (benzothienyl-2- and 3-yl)alanine, β-(1-and 2-naphthyl)alanine, O-alkylated derivatives of serine, threonine or tyrosine, S-alkylated cysteine, S-alkylated homocysteine, O- sulfate, O-phosphate and O-carboxylate esters of tyrosine, 3-(sulfo)tyrosine, 3- (carboxy)tyrosine, 3-(phospho)tyrosine, the 4-methane sulfonic acid ester of tyrosine, 4- methane phosphonic acid ester of tyrosine, 3,5-diiodotyrosine, 3-nitrotyrosine, ε-alkyl lysine, and δ-alkyl ornithine.

### Pharmaceutical compositions and kits

As used herein, a "pharmaceutical composition" refers to a preparation of one or more of the active ingredients described herein, with other components such as physiologically suitable carriers and excipients. The purpose of a pharmaceutical composition is to facilitate administration of a compound to a subject.

The pharmaceutical compositions of methods and compositions disclosed can be used in combination therapy with standard medicaments for the diseases listed herein above. A pharmaceutical composition may further comprise an inhibitor of cholesterol synthesis. Inhibitors of cholesterol synthesis are well known in the art, and include, e.g. statins. Statins alone are considered insufficient for treatment of Alzheimer's disease, since they inhibit cholesterol synthesis without actually stimulating egress of cholesterol from brain cells.

A pharmaceutical composition may further comprise a carrier, a solvent, an emulsifier, or enhancer of absorption.

Hereinafter, the phrases "therapeutically acceptable carrier" and "pharmaceutically acceptable carrier," which may be used interchangeably, refer to a carrier or a diluent that does not cause significant irritation to an organism and does not abrogate the biological activity and properties of the administered compound.

Herein, the term "excipient" refers to an inert substance added to a pharmaceutical composition to further facilitate administration of an active ingredient. Examples, without limitation, of particularly suitable excipients include calcium carbonate, calcium phosphate, various sugars and types of starch, cellulose derivatives, gelatin, oils, and polyethylene glycols.

A therapeutic composition may further comprise a pharmaceutically acceptable carrier. As used herein, a "carrier" refers to any substance suitable as a vehicle for delivering a therapeutic agent to a suitable *in vivo* site. As such, carriers can act as a pharmaceutically acceptable excipient of a therapeutic composition of the present invention. Examples of non-targeting carriers include, but are not limited to water, phosphate buffered saline, Ringer's solution, dextrose solution, serum-containing solutions, Hank's solution, other aqueous physiologically balanced solutions, oils, esters and glycols. Aqueous carriers can contain suitable auxiliary substances required to approximate the physiological conditions of the recipient, for example, by enhancing chemical stability and isotonicity.

Suitable auxiliary substances include, for example, sodium acetate, sodium chloride, sodium lactate, potassium chloride, calcium chloride, and other substances used to produce phosphate buffer, Tris buffer, and bicarbonate buffer. Auxiliary substances can also include preservatives, such as thimerosal, m- and o-cresol, formalin and benzol alcohol. Preferred auxiliary substances for aerosol delivery include surfactant substances non-toxic to a subject, for example, esters or partial esters of fatty acids containing from about six to about twenty-two carbon atoms. Other carriers can include metal particles (e.g., gold particles) for use with, for example, a biolistic gun through the skin. Therapeutic compositions of the present invention can be sterilized by conventional methods.

Targeting carriers are herein referred to as "delivery vehicles." Delivery vehicles are capable of delivering a therapeutic compound to a target site in a subject. A "target site" refers to a site in a subject to which one desires to deliver a therapeutic composition. Such target site may be the CNS of the subject, preferably the brain of the subject or the brain cells (e.g., astrocytes). Examples of delivery vehicles particularly suitable include, but are not limited to, artificial and natural lipid-containing delivery vehicles. Natural lipid-containing delivery vehicles include cells and cellular membranes. Artificial lipid-containing delivery vehicles include liposomes and micelles. A delivery vehicle of the present invention can be modified to target to a particular site in a subject, thereby targeting and making use of a therapeutic compound at that site. Suitable modifications include manipulating the chemical formula of the lipid portion of the delivery vehicle and/or introducing into the vehicle a compound capable of specifically targeting a delivery vehicle to a preferred site, for example, a preferred cell type. Specifically targeting refers to causing a delivery vehicle to bind to a particular cell by the interaction of the compound in the vehicle to a molecule on the surface of the cell. Suitable targeting compounds include ligands capable of selectively (i.e., specifically) binding another molecule at a particular site. Examples of such ligands include antibodies, antigens, receptors and receptor ligands. For example, an antibody specific for an antigen found on the surface of a target cell can be introduced to the outer surface of a liposome delivery vehicle so as to target the delivery vehicle to the target cell. Manipulating the chemical formula of the lipid portion of the delivery vehicle can modulate the extracellular or intracellular targeting of the delivery vehicle. For example, a chemical can be added to the lipid formula of a liposome that alters the charge of the lipid bilayer of the liposome so that the liposome fuses with particular cells having particular charge characteristics.

A delivery vehicle particularly suitable is a liposome. A liposome is capable of remaining stable in a subject for a sufficient amount of time to deliver a therapeutic compound of the present invention to a preferred site in the subject. A liposome is preferably stable in the subject into which it has been administered for at least about 30 minutes, more preferably for at least about 1 hour and even more preferably for at least about 24 hours.

Liposomes comprise liposome-forming lipids (also referred to as "vesicle-forming lipids") which are characterized by a hydrophilic tail and a hydrophobic and polar head group moiety, which can spontaneously form bilayer vesicles in water. Liposome-forming lipids are stably incorporated in lipid bilayers such that the hydrophobic moiety is in contact with the interior region of the vesicle membrane while the polar head group moiety is oriented to the exterior, polar surface of the vesicle membrane. Suitable liposomes for use with the present invention include any liposome. Preferred liposomes of the present invention include those liposomes commonly or routinely used in, for example, methods known to those of skill in the art. In certain embodiments, more preferred liposomes comprise liposomes having a polycationic lipid composition and/or liposomes having a cholesterol backbone conjugated to polyethylene glycol.

A variety of liposomal forming lipids can be used, as disclosed for example in U.S. Patent No. 5,919,480. The lipids or oily vesicle forming substances allow long-term storage of the liposome-encapsulated peptides and effective release of these components upon administration. Representative lipids include, but are not limited to, dimyristoyl phosphatidylcholine (DMPC), dimyristoyl phosphatidylglycerol (DMPG), cholesterol, 1,2-distearoyl-3-trimethylammonium propane (DSTAP), 1,2-dimyristoyl-3-trimethylammonium propane (DMTAP), and combinations thereof, such as DMPC/cholesterol, DMPC/DMPG, DMPC/DMPG/cholesterol, DMPC/DMTAP, and DMPG/DMTAP/cholesterol.

Liposomes may be prepared by a variety of techniques. To form multilamellar vesicles (MLV), a mixture of vesicle-forming lipids is dissolved in a suitable organic solvent (or solvent mixtures), for example tert-butanol, and evaporated in a vessel to form a thin film, which is then hydrated by an aqueous medium to form lipid vesicles, typically in sizes ranging from about 0.1 to about 10 µm. The lyophilized MLV preparation can be resolubilized as an aqueous suspension. The MLV suspension can then be selectively downsized to a desired vesicle size range e.g. 1 micron or less, by extruding aqueous suspension through a polycarbonate membrane having a select uniform pore size, typically 0.05 to 1.0 microns.

Phospholipids may spontaneously form vesicles in an aqueous environment or are stably incorporated into lipid bilayer membranes with the hydrophobic portion of the lipid molecule in the interior and the polar head group portion of the lipid molecule in the hydrophilic, external surface of the bilayer vesicle. The lipid bilayer membrane of the liposomal vesicle is suitable for holding the hydrophilic peptides within and on the lipoid membrane vesicle enclosure.

Vesicle-forming lipids may include hydrocarbon chains, a steroid group, or a chemically reactive group, such as acid, alcohol, aldehyde, amine or ester, as a polar head group. The phospholipids include vesicle forming combinations of phosphatidic acid (PA), phosphatidyl choline (PC), phosphatidyl ethanolamine (PE), phosphatidyl glycerol, phosphatidyl inositol (PI), and sphingomyelin (SM) which generally comprise two hydrocarbon chains of about 14-22 carbons at varying degrees of unsaturation. Lipopolymers can be added to stabilize the lipid content of the vesicles. Furthermore, vesicles can be formed from glycolipids, including cerebrosides and gangliosides, as well as sterols (i.e. cholesterol). Synthetic membrane forming phosphatidyl derivative compounds containing dihexadecyl, dioleoyl, dilauryl, dimyristoyl, or dipalmitoyl groups are also available (Calbiochem), including dimyristoyl phosphatidyl choline or dimyristoyl phosphatidyl glycerol which can be taken as a mixture, with and without lipid membrane stabilizing additives.

Various methods are available for encapsulating agents in the liposomes. For example, in the reverse phase evaporation method (Szoka, U.S. Patent No. 4,235,871) a non-aqueous solution of vesicle-forming lipids is dispersed with a smaller volume of an aqueous medium to form a water-in-oil emulsion. Thus, for encapsulation the active ingredients or agents are included either in the lipid solution, in the case of a lipophilic agent, or in the aqueous medium, as in the case of a water-soluble agent. After removal of the lipid solvent, the resulting gel is converted to liposomes. These reverse phase evaporation vesicles (REVs) have typical average sizes from about 2 to about 4 microns and are predominantly oligolamellar, that is, containing more than one or at least a few lipid bilayer shells. The REVs may be sized by extrusion, to give oligolamellar vesicles having e.g. a maximum selected size between about 0.05 and about 1.5 µm.

Preparations of large multilamellar vesicles (LMLV) or REV can be further treated, e.g., by way of extrusion, sonication or high pressure homogenization, to produce small unilamellar vesicles (SUV's), which are characterized by sizes in the range of about 0.03 micron to about 0.1 micron. Alternatively, SUV's can be formed directly by homogenization of an aqueous dispersion of lipids.

Other methods for adding additional components to liposomal compositions include methods wherein an aqueous liposome dispersion is co-lyophilized with other components and the resulting solid redispersed to form MLV. Another method (A. Adler, Cancer Biother. 10: 293, 1995) provides addition of an aqueous solution of the agent to be encapsulated to a t-butanol solution of lipids. The mixture is sonicated and lyophilized, and the resulting powder is rehydrated.

Liposome compositions containing an entrapped agent can again be treated after final sizing, if necessary, to remove the free (non-entrapped) agent. Conventional separation techniques, such as centrifugation, diafiltration, and ultrafiltration are suitable for this purpose.

The composition can also be sterilized by filtration through a conventional 0.45 micron filter. In order to form the compositions of the current invention, the concentration of peptides in the liposomes can be chosen to give a protein/lipid molar ratio from about 1:100 to about 1:1000, at 100% encapsulation, after filtration.

Stabilizers may also be added to the liposomal compositions, for example, a metal chelator. For more extended storage, the compositions may be converted to a dry lyophilized powder, and can be hydrated to form an aqueous suspension as needed before use.

The composition may further comprise micelles. As used herein, "micelles" refers to any water soluble aggregate which is spontaneously and reversibly formed from amphiphilic compounds or ions. Lipids, in particular phosphatidyl glycerol lipid derivatives are useful for forming micelles. Representative examples include dipalmitoyl phosphatidyl glycerol, dimyristoyl phosphatidyl glycerol, and dicapryl phosphatidyl glycerol. Micelles suitable for use in the invention are disclosed for example in US Patent Nos. 7,332,527 and 7,413,538.

### Therapeutic use

Disclosed is a method for treating, preventing, or inhibiting progression of a brain disease characterized by amyloid plaque deposits in a subject in need thereof, the method comprising the step of administering to the subject a therapeutically effective amounts of a therapeutic composition comprising FABAC, thereby treating, preventing, or inhibiting progression of a brain disease characterized by amyloid plaque deposits in a subject.

The subject may be a human or a non-human mammal.

Disclosed is the use of a bile acid fatty acid conjugate or bile salt fatty acid conjugate (FABAC) in the preparation of a medicament for treatment, prevention, or inhibition of progression of a brain disease characterized by amyloid plaque deposits.

The brain disease treated by is, in one embodiment, Alzheimer's disease. In another embodiment, the brain disease is any other brain disease characterized by amyloid plaque deposits.

### Alzheimer's disease

As used herein, the terms "Alzheimer's disease" and "AD" refer to a neurodegenerative disorder and encompass familial Alzheimer's disease and sporadic Alzheimer's disease. The term "familial Alzheimer's disease" refers to Alzheimer's disease associated with genetic factors (i.e., inheritance is demonstrated) while "sporadic Alzheimer's disease" refers to Alzheimer's disease that is not associated with prior family history of the disease. Symptoms indicative of Alzheimer's disease in human subjects typically include, but are not limited to, mild to severe dementia, progressive impairment of memory (ranging from mild forgetfulness to disorientation and severe memory loss), poor visual spatial skills, personality changes, poor impulse control, poor judgment, distrust of others, increased stubbornness, restlessness, poor planning ability, poor decision making, and social withdrawal. In severe cases, patients lose the ability to use language and communicate, and require assistance in personal hygiene, eating and dressing, and are eventually bedridden. Hallmark pathologies within brain tissue include extracellular neuritic β-amyloid plaques, neurofibrillary tangles, neurofibrillary degeneration, granulovascular neuronal degeneration, synaptic loss, and extensive neuronal cell death.

Alzheimer's Disease is both clinically and histopathologically heterogeneous. In less than 5% of cases the disease co-segregates with certain mutations of β-amyloid precursor protein, presenilin-1 or presenilin-2 genes. The remaining over 95% of AD cases are not associated with any known mutations and the nature of the etiological agent(s), and/or predisposing factor(s), are not yet understood. Independent of the etiology, whether genetic or non-genetic, Alzheimer's Disease is characterized clinically by progressive dementia, and histopathologically by the presence of neurofibrillary tangles and neuritic amyloid plaques with neurofibrillary changes in the dystrophic neurites. Because of its clinical heterogeneity, the diagnosis of Alzheimer's Disease remains probable till postmortem histopathological examination of the brain, and is made using primarily criteria which exclude other causes of dementia.

Alzheimer's Disease is the most common type of progressive dementia in the elderly population, and constitutes an enormous unmet medical need due to its increasing prevalence in the aging Western world. Despite intense efforts, there are as yet no treatments which effectively target mechanisms leading to the formation of its pathological lesions.

In Alzheimer's Disease (AD), the abnormal cleavage of beta amyloid protein precursor (APP) from the intracellular membrane often produces a protein Abeta (e.g., Aβ 1-42) which is incompletely removed by normal clearance processes. It has been reported that soluble beta amyloid oligomers are highly neurotoxic. Moreover, over time, this soluble protein assemblage is deposited as a beta amyloid protein Abeta plaque within brain tissue, leading to the local destruction of neurons. The Abeta plaque deposition is also believed to provoke an inflammatory response by microglia and macrophages, which recognize the plaque as a foreign body. These cells are believed to respond to the plaque deposition by releasing pro-inflammatory cytokines and reactive oxygen species (ROS). Although the inflammatory response may be provoked in an effort to clear the brain tissue of the detrimental plaque, it is now believed that this inflammation also injures local neuronal tissue, thereby exacerbating AD.

As used herein "preventing, treating or inhibiting progression" includes abrogating, substantially inhibiting, slowing, reversing the progression, substantially ameliorating or substantially preventing clinical symptoms and/or pathological signs associated with Alzheimer's Disease, whether in a human subject or an animal model of Alzheimer's Disease.

The disclosed methods may be applied to subjects with a predisposition for Alzheimer's Disease (e.g., having a known genetic risk of developing AD). The methods may further comprise the step of identifying a predisposition for Alzheimer's Disease in a subject. As used herein "predisposition" refers to a state of having a relatively increased tendency or susceptibility to contracting or developing a particular condition or disease. Accordingly "a predisposition for Alzheimer's Disease" refers to the state of having an increased tendency or susceptibility for developing Alzheimer's Disease, as compared to other individuals lacking the predisoposition. A predisposition for Alzheimer's Disease may be the result of innate genetic factors, such as a specific genetic mutation, or it may be the result of an earlier injury (for example, brain trauma), infection with a particular etiologic agent, or a pre-existing underlying condition (for example, Down's syndrome).

Individuals having a known genetic risk of developing AD include those having relatives afflicted with AD, and those whose risk is determined by analysis of genetic or biochemical markers. Genetic markers of risk toward AD include mutations in the APP gene, particularly mutations at position 717 and positions 670 and 671 referred to as the Hardy and Swedish mutations respectively (see Hardy et al., 1997, Trends Neurosci 20, 154-159). Other markers of risk are mutations in the presenilin genes, PS1 and PS2, and ApoE4. Individuals presently suffering from AD can be recognized from characteristic dementia, as well as the presence of risk factors described above. In addition, a number of known in the art diagnostic tests are available for identifying individuals who have AD. These involve measurement of CSF tau and Aβ1-42 levels.

### Brain diseases characterized by amyloid plaque deposits

Though commonly associated with AD, amyloid plaque deposits are present in several other CNS diseases or disorders. Non-limiting example of such CNS diseases or disorders comprise: Down's syndrome, mild cognitive impairment (MCI), senile dementia and cerebral amyloid angiopathy (CAA).

Thus, the methods and compositions disclosed may also be applied to subjects having Down's syndrome, since these individuals are at risk of developing AD-like neurological manifestations with age. They may also be applied to subjects having MCI. Thus, they may also be applied to subjects having senile dementia.

Cerebral amyloid angiopathy (CAA), also known as cerebrovascular amyloidosis, is a disease of small blood vessels in the brain in which deposits of amyloid protein in the vessel walls may lead to stroke, brain hemorrhage, or dementia. CAA is commonly associated with Alzheimer's disease, Down's syndrome and normal aging, as well as with a variety of familial conditions related to stroke or dementia (see Frangione et al., Amyloid: J. Protein Folding Disord. 8, Suppl. 1, 36-42 (2001)). CAA can occur sporadically or be hereditary. By virtue of FABACs effect on amyloid plaque deposits in the CNS, the methods and compositions of the invention may also be applied to subjects having CAA.

The methods disclosed may further comprise assessing the level of cognitive function in the subject prior to and following the step of administering the composition, wherein an improvement in the level of cognitive function is indicative of the efficacy of the method.

Additional diagnostic tests are available for identifying individuals who have AD, e.g., measurement of CSF tau and Aβ1-42 levels. Thus, the methods of the present invention may further involve assaying the level of anti-Aβ antibodies (e.g., Aβ1-42) in the serum of the subject prior to and following administration of the composition. Conveniently, the assaying may comprise use of an ELISA technique, or any other similar technique known in the art to identify and quantify serum antibodies.

For example, in a suitable ELISA technique, Aβ1-42 is coated onto the walls of 96-well microtiter plates. Serial dilutions of serum obtained from a subject are added to the wells and incubated under appropriate conditions to allow specific antigen-antibody binding. The reacted serum is washed away and a first known antibody having specificity for the Fc portion of human IgG, e.g. goat anti-human IgG, conjugated with an enzyme (e.g. horseradish peroxidase, alkaline phosphatase, glucose oxidase, or β-galactosidase) is added to the wells and incubated under appropriate conditions to allow specific binding. After washing, appropriate enzyme substrate is added and the colorometric reaction is quantified in a dedicated sample reader. For a more sensitive sandwich assay, the enzyme may be conjugated to a second known antibody having specificity for the first known antibody.

The term "therapeutically effective amounts" referred to herein means that the compounds or compositions of the invention are administered to the subject in amounts that are effective to treat that subject. A therapeutically effective amount may vary among individuals, depending upon many different factors, including means of administration, target site, physiological state of the patient, the gender of the subject, other medications administered, and whether treatment is prophylactic or therapeutic. The subject is generally a human, but may also be a nonhuman mammal. The composition is typically administered in several dosages over prolonged periods until a sufficient response has been achieved.

The term "patient" includes human subjects that are diagnosed to suffer from Alzheimer's disease.

An effective administration protocol (i.e., administering a pharmaceutical composition in an effective manner) comprises suitable dose parameters and modes of administration that result in treatment of a disease. Effective dose parameters and modes of administration can be determined using methods standard in the art for a particular disease. Such methods include, for example, determination of survival rates, side effects (i.e., toxicity) and progression or regression of disease.

In accordance with the present invention, a suitable single dose size is a dose that is capable of treating a subject with disease when administered one or more times over a suitable time period.

Preferably, compositions of the present invention are administered orally. Other suitable routes of administration may, for example, include rectal, transmucosal, especially transnasal, intestinal or parenteral delivery.

Alternately, one may administer a preparation in a local rather than systemic manner, for example, via injection of the preparation directly into a specific region of a patient's body, such as the liver, or by direct administration into a body cavity such as the gall bladder or ventricle.

Solutions or suspensions used for parenteral, intradermal, or subcutaneous application can include the following components: a sterile diluent such as water for injection, saline solution, fixed oils, polyethylene glycols, glycerine, propylene glycol (or other synthetic solvents), antibacterial agents (e.g., benzyl alcohol, methyl parabens), antioxidants (e.g., ascorbic acid, sodium bisulfite), chelating agents (e.g., ethylenediaminetetraacetic acid), buffers (e.g., acetates, citrates, phosphates), and agents that adjust tonicity (e.g., sodium chloride, dextrose). The pH can be adjusted with acids or bases, such as hydrochloric acid or sodium hydroxide, for example. The parenteral preparation can be enclosed in ampules, disposable syringes or multiple dose vials made of glass or plastic.

Pharmaceutical compositions adapted for parenteral administration include, but are not limited to, aqueous and non-aqueous sterile injectable solutions or suspensions, which can contain antioxidants, buffers, bacteriostats and solutes that render the compositions substantially isotonic with the blood of an intended recipient. Such compositions can also comprise water, alcohols, polyols, glycerine and vegetable oils, for example. Extemporaneous injection solutions and suspensions can be prepared from sterile powders, granules and tablets. Such compositions should comprise a therapeutically effective amount of a vector of the invention and/or other therapeutic agent, together with a suitable amount of carrier so as to provide the form for proper administration to the subject. The formulation should suit the mode of administration.

The disclosed compounds or compositions may be provided in packs in a form ready for administration or in concentrated form in packs, optionally with the diluent required to make final solution(s) for administration. A product may contain a compound useful in the invention in solid form and, optionally, a separate container with a suitable solvent or carrier for the compound useful in the invention.

The packs/kits may include other components, e.g., instructions for dilution, mixing and/or administration of the product, other containers, syringes, needles, etc. Other such pack/kit components will be readily apparent to one of skill in the art.

The kits may further comprise instructions for administering the compound or composition.

The following examples are presented in order to more fully illustrate some embodiments of the invention. They should, in no way be construed, however, as limiting the broad scope of the invention.

### EXAMPLES

### EXAMPLE 1

### FABACs cross the blood-brain barrier in rats

Male Wistar rats weighing 200-250 gm received a single gavage administration of ¹⁴C-labeled-Aramchol (suspended in 0.3 ml saline), at a target dose level of 16µCi/ml (40µCi/ kg). Aramchol is also known as "3beta-arachidyl amido, 7α,12α-dihydroxy-5-beta-cholan-24-oic acid" or "C-20 FABAC," an amide conjugate (at position 3 of the bile acid) of arachidic and cholic acids. Groups of 3 animals were sacrificed following gavage, using ketamine anesthesia, at 3, 12, 24 or 72 hours.

Brain tissue samples were excised, homogenized in ice-cold 5M guanidine thiocyanate/HCl, pH 8, and rotated at room temperature for 4 h. Homogenates were stored at -80°C. Brain homogenates were diluted and centrifuged, and the ¹⁴C-label was detected by mixing samples with scintillation fluid and reading in a liquid scintillation counter.

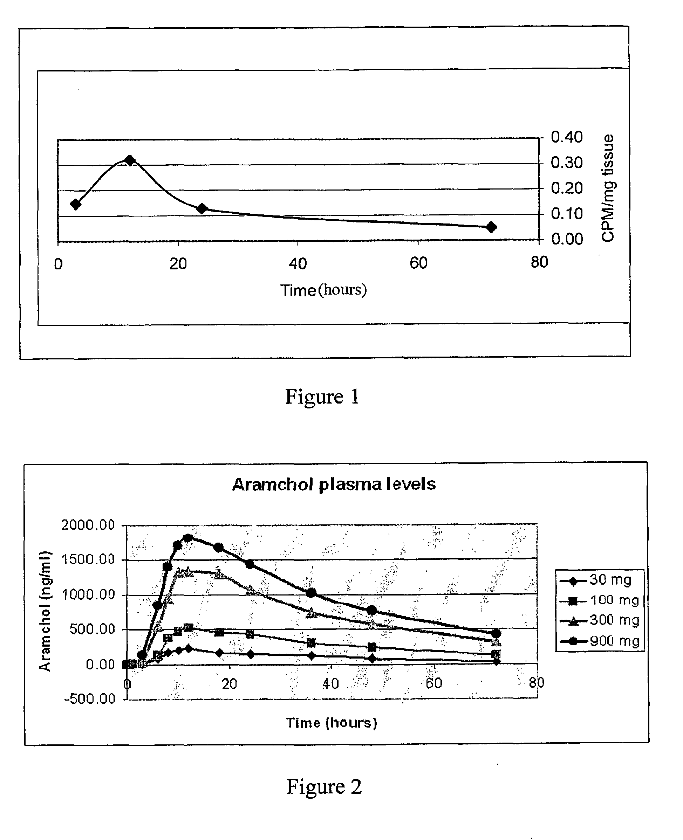

Labeled Aramchol was administered orally to rats, to determine whether it could cross the blood-brain barrier. Radioactivity in brain tissues was detectable, but levels were not high. Radioactivity was first detected at 3 hours, rose to a peak at 12 hours, and disappeared towards 24 hours. The signal increased between the 3-and 12-hr time points from 0.15 cpm/mg tissue to 0.32 cpm/mg/tissue (Figure **1**).

**Figure 1** shows that FABACs cross the brain blood barrier in rats.

### EXAMPLE 2

### Aramchol plasma levels over time in normal human subjects following oral administration of ascending doses

To 200 µL of each plasma sample, calibrator, and QC sample were added 20 µL of 2.5 µg/mL internal standard solution in 1:1 methanol:water followed by 50 µL of 5% acetic acid and 2 mL of methyltertbutylether. The samples were mixed on a rotating shaker for 5 minutes and centrifuged. After centrifugation, the aqueous layer of the samples was frozen and the upper (organic) layer was transferred to evaporation tubes and evaporated to dryness under a gentle stream of N₂ at ~40°C. The samples were then reconstituted in 200 µL of 5:4:1 methanol:acetonitrile:20mM ammonium acetate, mixed, and centrifuged. 100 µL of the clear upper solution was transferred to auto-sampler vials and analyzed by UPLC-MS/MS using a Waters Acquity UPLC BEH C18, 50x2.1 mm, 1.7µ column. A sample blank (blank plasma) and reagent blank (water) were also prepared, but without internal standard added (1:1 methanol:water added instead).

Normal human volunteers (n=12) ingested Aramchol in a formulation containing sodium lauryl sulfate and carboxy methyl cellulose, in a single dose containing 30, 100, 300, or 900mg of Aramchol. Blood was sampled at 0, 1, 3, 4, 6, 9, 12, 18, 24, 36, 48, and 72 hours following ingestion. Aramchol blood levels were measured by a mass spectroscopy-based method (Goldiner et al., J Chromatogr B Analyt Technol Biomed Life Sci 2003;795: 35-40). Results are shown in Figure 2. Aramchol plasma levels were measurable at all doses and were dose-proportional. The Cₘₐₓ was around 12 hours. Importantly, high Aramchol levels were present at 24 hr and substantial levels at 36 hr, following a single ingestion.

### EXAMPLE 3

### Effects of FABACs on cholesterol efflux from a human astrocyte cell line

Human astrocyte cells (1 x10⁶ cells/ml; ccf-sttg1 cell line) were incubated in 24 plates for 24-48 hours in 10% FCS (Fetal Calf Serum) in DMEM (Dulbecco's modified Eagle's Media) in a humidified 37 °C incubator in the presence of 7.5% CO₂. After the incubation time Cholesterol loading was performed by incubating the cells for 20 hr with DMEM supplemented with 30 µg/ml cholesterol, 0.5 µCi/ml of [³H]cholesterol and 0.2% fatty-acid-free BSA (Bovine Serum Albumin). After incubation, the cells were washed four times with PBS (Phosphate Buffered Saline) containing 0.2% fatty-acid-free BSA. Cells were then incubated for 20hr in a humidified incubator at 37 °C in the presence of 10% CO₂, with or without apolipoproteins (ApoE) or FABACs (Aramchol or Steamchol) at several concentrations. The medium was collected and centrifuged at 12000 *g* for 5 min to remove cellular debris. The aliquots of the supernatant were taken to count the effluxed [³H]cholesterol. The remaining cell-associated [³H]cholesterol was determined after extraction for at least 30 min with propan-2-ol. Efflux was determined in the presence, or absence, of apolipoproteins or FABACs. FABACs were dissolved in ethanol and added to the medium at the concentrations indicated. The amount of [³H]cholesterol in the medium and cellular extract was quantified by liquid-scintillation counting. The radioactivity released into the medium was expressed as the fraction of the total radioactive cholesterol that was present in the supernatant of each well. The percentage efflux was calculated by dividing the radioactive counts in the efflux medium by the sum of the counts in the medium and cell extract.

Aramchol increased cholesterol efflux from human astrocyte brain cells (ccf-sttg1 cell line). The maximal effect of Aramchol was reached at concentrations 5-10µg/ml Aramchol. Lower efflux levels were demonstrated at a concentration of 2µg/ml Aramchol. Higher Aramchol concentrations (20 µg/ml) showed a decrease in cholesterol efflux.

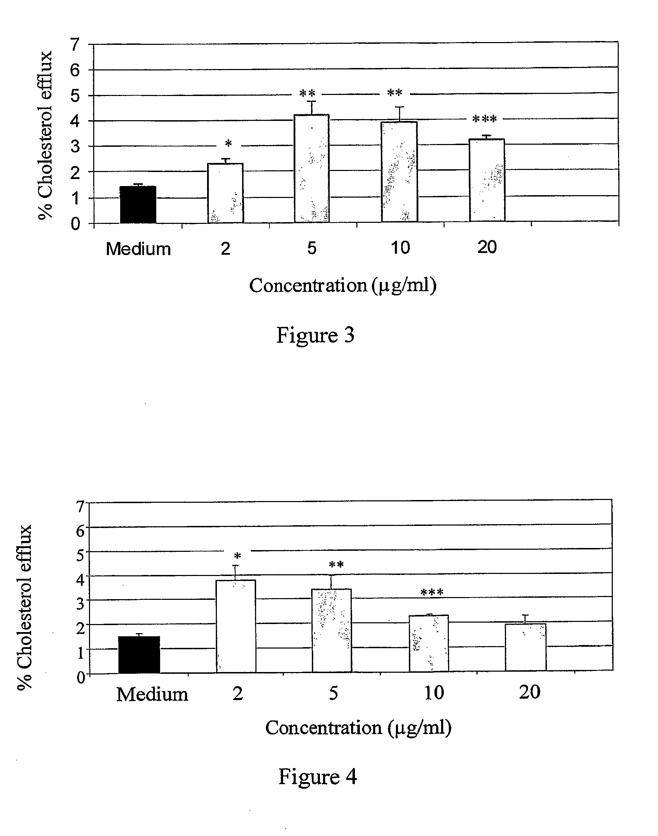

**Figure 3** shows the direct effect of a FABAC molecule, Aramchol, on cholesterol efflux from astrocyte brain cells.

Steamchol (3β-stearoylamido-7α,12α-dihydroxy-5β-cholan-24-oic acid), however, showed higher potency than Aramchol by evoking a maximal efflux at the low concentration of 2 micrograms/ml.

**Figure 4** shows the direct effect of a FABAC molecule, Steamchol, on cholesterol efflux from astrocyte brain cells. Advantageously, said effect was achieved at low concentrations.

### EXAMPLE 4

### Effects of brain apolipoproteins (ApoE3 and ApoE4) on cholesterol efflux from a human astrocyte cell line

Astrocyte brain cells (ccf-sttg1 cell line) were maintained under the same conditions as above (Example 3) and the effect of the 2 physiological stimulants ApoE3 and ApoE4 was tested. Both brain lipoproteins evoked cholesterol efflux from the cells, demonstrating that the experimental setup is physiologic. Unexpectedly, the FABACs were more potent than the 2 tested brain apolipoproteins. FABACs evoked a maximal cholesterol efflux at the concentrations of 2 microgram/ml (Steamchol) and 5 microgram/ml (Aramchol) while the apolipoproteins reached a maximal effect at the concentration of 10 microgram/ml. Furthermore, at the above concentrations, the % of cell cholesterol removed into the medium was about 25% higher with the FABACs as compared to the apolipoproteins. Note that the control efflux induced by the medium only is equal in both sets of experiments. The efflux at concentrations of 20 microgram/ml is problematic, since cell damage occurs at this concentration.

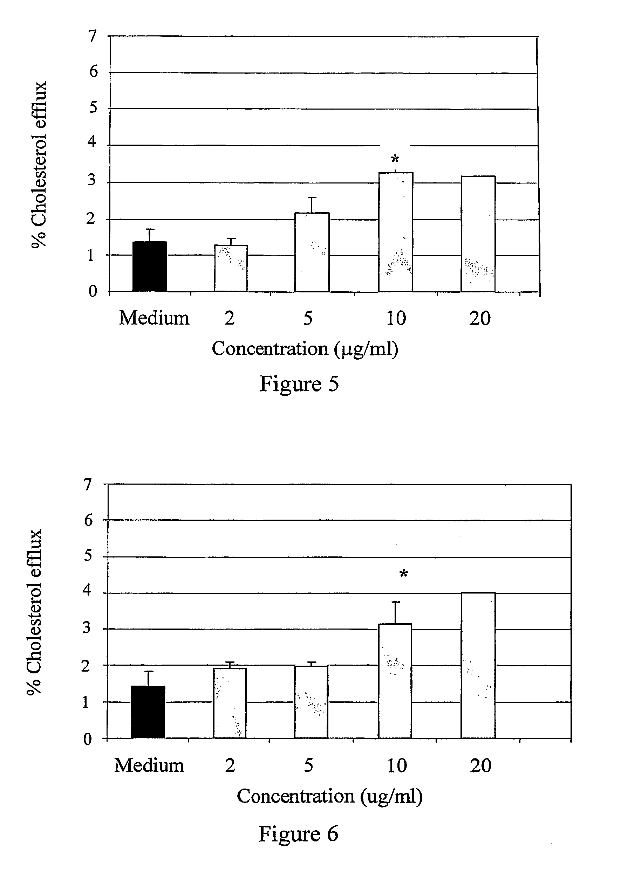

**Figures 5 - 6** show the effect of ApoE3 and ApoE4, respectively, on cholesterol efflux from astrocyte brain cells.

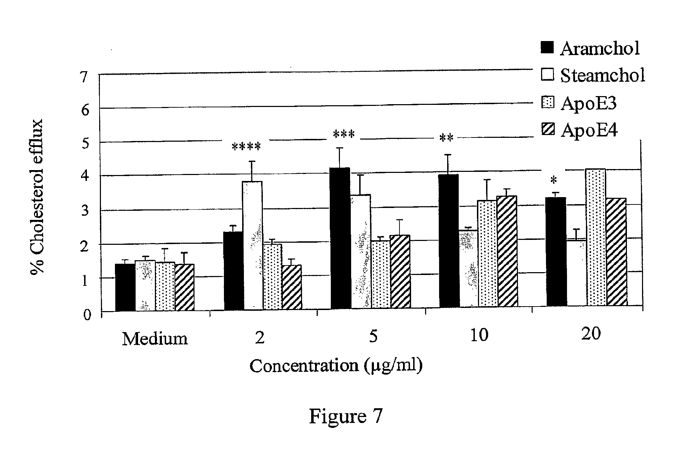

**Fig.7** summarizes the results and illustrates that FABACs, particularly 3β-stearoylamido-7α,12α-dihydroxy-5β-cholan-24-oic acid (Steamchol) have a greater effect on cholesterol efflux from human astrocytes, than the human Apolipoproteins ApoE3 and ApoE4.

## Claims

1. A pharmaceutical composition comprising as an active ingredient a bile acid fatty acid conjugate or bile salt fatty acid conjugate (FABAC), for use in the treatment, prevention, or inhibition of progression of a brain disease **characterized by** amyloid plaque deposits, wherein said FABAC has the formula II:
W-X-G (II)
wherein G represents a bile acid or bile salt;
W represents one or two saturated or unsaturated fatty acids having 14-22 carbon atoms;
and X represents an NH bonding member, and wherein said brain disease is selected from the group consisting of Alzheimer's disease, Down's syndrome, mild cognitive impairment, senile dementia and cerebral amyloid angiopathy.

2. The composition for use of claim 1, wherein said brain disease is Alzheimer's disease.

3. The composition for use of claim 1 comprising two fatty acids wherein at each occurrence W is independently a saturated or unsaturated fatty acid having 14-22 carbon atoms.

4. The composition for use of any of claims 1 or 3,
wherein said fatty acids are independently selected from the group consisting of behenylic acid, arachidylic acid, stearic acid, and palmitic acid; or
wherein said bile acid is selected from the group consisting of cholic acid, ursodeoxycholic acid, chenodeoxycholic acid, deoxycholic acid, lithocholic acid, and derivatives thereof; or
wherein said bile acid or bile salt is conjugated to an amino acid.

5. The composition for use of claim 1,
wherein said FABAC is selected from the group consisting of 3-beta-stearoyl-amido,7α,12α-dihydroxy-5-beta-cholan-24-oic acid and 3-beta arachidylamido,7α,12α-dihydroxy-5-beta-cholan-24-oic acid; or
wherein said composition further comprises an inhibitor of cholesterol synthesis.

6. A bile acid fatty acid conjugate or bile salt fatty acid conjugate (FABAC) for use in the treament, prevention, or inhibition progression of a brain disease, said brain disease being **characterized by** amyloid plaque, wherein said FABAC has the formula II:
W-X-G (II)
wherein G represents a bile acid or bile salt; W represents one or two saturated or unsaturated fatty acids having 14-22 carbon atoms; and X represents an NH bonding member, and wherein said brain disease is selected from the group consisting of Alzheimer's disease, Down's syndrome, mild cognitive impairment, senile dementia and cerebral amyloid angiopathy.

7. The bile acid fatty acid conjugate or bile salt fatty acid conjugate (FABAC) for use of claim 6, wherein said brain disease is Alzheimer's disease.

8. The bile acid fatty acid conjugate or bile salt fatty acid conjugate (FABAC) for use of claim 6, wherein the FABAC comprises two fatty acids wherein at each occurrence W is independently a saturated or unsaturated fatty acid having 14-22 carbon atoms.

9. The bile acid fatty acid conjugate or bile salt fatty acid conjugate (FABAC) for use of any of claims 6 or 8,
wherein said fatty acids are independently selected from the group consisting of behenylic acid, arachidylic acid, stearic acid, and palmitic acid; or
wherein said bile acid is selected from the group consisting of cholic acid, ursodeoxycholic acid, chenodeoxycholic acid, deoxycholic acid, lithocholic acid, and derivatives thereof; or
wherein said bile acid or bile salt is conjugated to an amino acid; or
wherein said FABAC is selected from the group consisting of 3-beta-stearoyl-amido,7α,12α-dihydroxy-5-beta-cholan-24-oic acid and 3-beta arachidylamido,7α,12α-dihydroxy-5-beta-cholan-24-oic acid.

10. The bile acid fatty acid conjugate or bile salt fatty acid conjugate (FABAC) for use of claim 6, wherein said FABAC is administered in a pharmaceutical composition that further comprises an inhibitor of cholesterol synthesis.

11. The bile acid fatty acid conjugate or bile salt fatty acid conjugate (FABAC) for use according to any of claims 6, 8-10, wherein said brain disease is selected from the group consisting of Alzheimer's disease, Down's syndrome, mild cognitive impairment, senile dementia and cerebral amyloid angiopathy.

## Patentansprüche

1. Pharmazeutische Zusammensetzung umfassend als einen aktiven Bestandteil ein Gallensäure-Fettsäure Konjugat oder Gallensalz-Fettsäure Konjugat (FABAC) zur Verwendung in der Behandlung, Prävention oder Hemmung des Fortschreitens einer Gehirnerkrankung **gekennzeichnet durch** Ablagerungen von Amyloid-Plaques, wobei das FABAC, die Formel II aufweist:
W-X-G (II)
worin G für eine Gallensäure oder ein Gallensalz steht.
W für eine oder zwei gesättigte oder ungesättigte Fettsäuren mit 14-22 Kohlenstoffatomen steht, und
X für ein NH-Bindungselement steht, und wobei die Gehirnerkrankung ausgewählt ist aus der Gruppe bestehend aus Alzheimer, Down-Syndrom, leichte kognitive Störung, senile Demenz und zerebraler Amyloidangiopathie.

2. Zusammensetzung zur Verwendung nach Anspruch 1, wobei die Gehirnerkrankung Alzheimer ist.

3. Zusammensetzung zur Verwendung nach Anspruch 1, umfassend zwei Fettsäuren, wobei bei jedem Auftreten W unabhängig eine gesättigte oder ungesättigte Fettsäure mit 14-22 Kohlenstoffatomen ist.

4. Zusammensetzung zur Verwendung nach einem der Ansprüche 1 oder 3,
wobei die Fettsäuren unabhängig voneinander ausgewählt sind aus der Gruppe bestehend aus Behensäure, Arachidylsäure, Stearinsäure und Palmitinsäure, oder
wobei die Gallensäure ausgewählt ist aus der Gruppe bestehend aus Cholsäure, Ursodesoxycholsäure, Chenodesoxycholsäure, Desoxycholsäure, Lithocholsäure und Derivaten davon, oder
wobei die Gallensäure oder das Gallensäuresalz an einer Aminosäure konjugiert vorliegt.

5. Zusammensetzung zur Verwendung nach Anspruch 1.
wobei das FABAC ausgewählt ist aus der Gruppe bestehend aus 3-Beta-Stearoyl-amido,7α,12α-dihydroxy-5-beta-cholan-24-olsäure und 3-Beta-Arachidylamido,7α,12α-dihydroxy-5-beta-cholan-24-olsäure, oder
wobei die Zusammensetzung ferner einen Inhibitor der Cholesterolsynthese umfasst.

6. Gallensäure-Fettsäure Konjugat oder Gallensalz-Fettsäure Konjugat (FABAC) zur Verwendung in der Behandlung, Prävention oder Hemmung des Fortschreitens einer Gehirnerkrankung, wobei die Gehirnerkrankung **gekennzeichnet ist durch** Amyloid-Plaques, wobei das FABAC die Formen II aufweist:
W-X-G (II)
worin G für eine Gallensäure oder ein Gallensalz steht.
W für eine oder zwei gesättigte oder ungesättigte Fettsäuren mit 14-22 Kohlenstoffatomen steht, und
X für ein NH-Bindungselement steht, und wobei die Gehirnerkrankung ausgewählt ist aus der Gruppe bestehend aus Alzheimer, Down-Syndrom, leichte kognitive Störung, senile Demenz und zerebraler Amyloidangiopathie.

7. Das Gallensäure-Fettsäure Konjugat oder Gallensalz-Fettsäure Konjugat (FABAC) zur Verwendung nach Anspruch 6, wobei die Gehirnerkrankung Alzheimer ist.

8. Das Gallensäure-Fettsäure Konjugat oder Gallensalz-Fettsäure Konjugat (FABAC) zur Verwendung nach Anspruch 6, wobei das FABAC zwei Fettsäuren umfasst, wobei bei jedem Auftreten W unabhängig eine gesättigte oder ungesättigte Fettsäure mit 14-22 Kohlenstoffatomen ist.

9. Das Gallensäure-Fettsäure Konjugat oder Gallensalz-Fettsäure Konjugat (FABAC) zur Verwendung nach einem der Ansprüche 6 oder 8,
wobei die Fettsäuren unabhängig voneinander ausgewählt sind aus der Gruppe bestehend aus Behensäure, Arachidylsäure, Stearinsäure und Palmitinsäure, oder
wobei die Gallensäure ausgewählt ist aus der Gruppe bestehend aus Cholsäure. Ursodesoxycholsäure. Chenodesoxycholsäure. Desoxycholsäure, Lithocholsäure und Derivaten davon, oder
wobei die Gallensäure oder das Gallensäuresalz an einer Aminosäure konjugiert vorliegt, oder
wobei das FABAC ausgewählt ist aus der Gruppe bestehend aus 3-Beta-Stearoyl-amido,7α,12α-dihydroxy-5-beta-choian-24-olsäure und 3-Beta-Arachidylimido,7α,12α-dihydroxy-5-beta-cholan-24-olsäure, oder
wobei die Zusammensetzung ferner einen Inhibitor der Cholesterolsynthese umfasst.

10. Das Gallensäure-Fettsäure Konjugat oder Gallensalz-Fettsäure Konjugat (FABAC) zur Verwendung nach Anspruch 6, wobei das FABAC in einer pharmazeutischen Zusammensetzung verabreicht wird, die ferner einen Inhibitor der Cholesterolsynthese umfasst.

11. Das Gallensäure-Fettsäure Konjugat oder Gallensalz-Fettsäure Konjugat (FABAC) zur Verwendung nach Anspruch 6 oder 8-10, wobei die Gehirnerkrankung ausgewählt ist aus der Gruppe bestehend aus Alzheimer, Down-Syndrom, leichte kognitive Störung, senile Demenz und zerebraler Amyloidangiopathie.

## Revendications

1. Composition pharmaceutique comprenant comme principe actif un conjugué acide biliaire-acide gras ou un conjugué sel biliaire-acide gras (FABAC), pour une utilisation dans le traitement, la prévention ou l'inhibition de la progression d'une maladie cérébrale **caractérisée par** des dépôts de plaques amyloïdes, ledit FABAC présentant la formule II :
W - X - G (II)
dans laquelle G représente un acide biliaire ou sel biliaire ;
W représente un ou deux acides gras saturés ou insaturés comprenant 14-22 atomes de carbone ;
et X représente un élément de liaison NH, et ladite maladie cérébrale étant choisie dans le groupe constitué par la maladie d'Alzheimer, le syndrome de Down, un trouble cognitive léger, la démence sénile et l'angiopathie amyloïde cérébrale.

2. Composition pour une utilisation selon la revendication 1, ladite maladie cérébrale étant la maladie d'Alzheimer.

3. Composition pour une utilisation selon la revendication 1, comprenant deux acides gras, W représentant indépendamment, à chaque occurrence, un acide gras saturé ou insaturé comprenant 14-22 atomes de carbone.

4. Composition pour une utilisation de l'une quelconque des revendications 1 ou 3,
lesdits acides gras étant indépendamment choisis dans le groupe constitué par l'acide béhénylique, l'acide arachidique, l'acide stéarique et l'acide palmitique ; ou ledit acide biliaire étant choisi dans le groupe constitué par l'acide cholique, l'acide ursodésoxycholique, l'acide chénodésoxycholique, l'acide désoxycholique, l'acide lithocholique et leurs dérivés ; ou
ledit acide biliaire ou sel biliaire étant conjugué à un acide aminé.

5. Composition pour une utilisation selon la revendication 1,
ledit FABAC étant choisi dans le groupe constitué par l'acide 3-bêta-stéaroylamido-7α,12α-dihydroxy-5-bêta-cholan-24-oïque et l'acide 3-bêta arachidylamido-7α,12α-dihydroxy-5-bêta-cholan-24-oïque ; ou
ladite composition comprenant en outre un inhibiteur de la synthèse de cholestérol.

6. Conjugué acide biliaire-acide gras ou conjugué sel biliaire-acide gras (FABAC) pour une utilisation dans le traitement, la prévention ou l'inhibition de la progression d'une maladie cérébrale, ladite maladie cérébrale étant **caractérisée par** des plaques amyloïdes, ledit FABAC présentant la formule II :
W-X-G (II)
dans laquelle G représente un acide biliaire ou sel biliaire ; W représente un ou deux acides gras saturés ou insaturés comprenant 14-22 atomes de carbone ; et X représente un élément de liaison NH, et ladite maladie cérébrale étant choisie dans le groupe constitué par la maladie d'Alzheimer, le syndrome de Down, un trouble cognitif léger, la démence sénile et l'angiopathie amyloïde cérébrale.

7. Conjugué acide biliaire-acide gras ou conjugué sel biliaire-acide gras (FABAC) pour une utilisation selon la revendication 6, ladite maladie cérébrale étant la maladie d'Alzheimer.

8. Conjugué acide biliaire-acide gras ou conjugué sel biliaire-acide gras (FABAC) pour une utilisation selon la revendication 6, le FABAC comprenant deux acides gras, W représentant indépendamment, à chaque occurrence, un acide gras saturé ou insaturé comprenant 14-22 atomes de carbone.

9. Conjugué acide biliaire-acide gras ou conjugué sel biliaire-acide gras (FABAC) pour une utilisation selon l'une quelconque des revendications 6 ou 8,
lesdits acides gras étant indépendamment choisis dans le groupe constitué par l'acide béhénylique, l'acide arachidique, l'acide stéarique et l'acide palmitique ; ou
ledit acide biliaire étant choisi dans le groupe constitué par l'acide cholique, l'acide ursodésoxycholique, l'acide chénodésoxycholique, l'acide désoxycholique, l'acide lithocholique et leurs dérivés ; ou
ledit acide biliaire ou sel biliaire étant conjugué à un acide aminé ; ou
ledit FABAC étant choisi dans le groupe constitué par l'acide 3-bêta-stéaroylamido-7α,12α-dihydroxy-5-bêta-cholan-24-oïque et l'acide 3-bêta arachidylamido-7α,12α-dihydroxy-5-bêta-cholan-24-oïque.

10. Conjugué acide biliaire-acide gras ou conjugué sel biliaire-acide gras (FABAC) pour une utilisation selon la revendication 6, ledit FABAC étant administré dans une composition pharmaceutique qui comprend en outre un inhibiteur de la synthèse de cholestérol.

11. Conjugué acide biliaire-acide gras ou conjugué sel biliaire-acide gras (FABAC) pour une utilisation selon l'une quelconque des revendications 6, 8-10, ladite maladie cérébrale étant choisie dans le groupe constitué par la maladie d'Alzheimer, le syndrome de Down, un trouble cognitif léger, la démence sénile et l'angiopathie amyloïde cérébrale.
